(19) Europäisches Patentamt
European Patent Office
Office européen des brevets

(11) Numéro de publication: **0 008 251**
**B1**

(12) **FASCICULE DE BREVET EUROPEEN**

(45) Date de publication du fascicule de brevet: **17.02.82**

(51) Int. Cl.³: **H 01 B 3/24,** C 07 C 25/18, C 07 C 17/26, H 01 F 27/32, H 01 G 4/22

(21) Numéro de dépôt: **79400478.8**

(22) Date de dépôt: **10.07.79**

(54) **Nouveaux diélectriques liquides, procédé de préparation et applications.**

(30) Priorité: **27.07.78 FR 7822216**
**20.02.79 FR 7904259**

(43) Date de publication de la demande:
**20.02.80 Bulletin 80/4**

(45) Mention de la délivrance du brevet:
**17.02.82 Bulletin 82/7**

(84) Etats contractants désignés:
**AT BE CH DE FR GB IT LU NL SE**

(56) Documents cités:
**BE-A-402 381**
**FR-A-931 987**
**FR-A-2 312 841**
**US-A-2 233 404**
**US-A-3 362 908**

**Le dossier contient des informations techniques présentées postérieurement au dépôt de la demande et ne figurant pas dans le présent fascicule.**

(73) Titulaire: **P C U K PRODUITS CHIMIQUES UGINE KUHLMANN, Service Propriété Industrielle Tour Manhattan, F-92087 PARIS LA DEFENSE 2 Cédex 21 (FR)**

(72) Inventeur: **Mathais, Henri, Le Cert - Champ sur Drac, F-38560 Jarrie (FR)**
Inventeur: **Commandeur, Raymond, Rue Marcel des Pres Avenue le Veneria, F-38220 Vizille (FR)**
Inventeur: **Pontoglio, Achille, Via Gabriele Rosa, 13, Brescia (IT)**
Inventeur: **Nebel, Sergio, Via Vincenzo Foppa, 3, Brescia (IT)**

EP 0 008 251 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Nouveaux diélectriques liquides, procédé de préparation et applications

La présente invention concerne de nouveaux diélectriques liquides à base de composés alkylaromatiques chlorés sur le noyau préparés à partir de chlorotoluènes, ou de chloroxylènes, ou de mélanges des deux.

Les produits à base de polychlorobiphényles ont été jusqu'ici largement utilisés dans le domaine de l'isolement des appareillages électriques car, outre leurs propriétés électriques, ils réunissent un certain nombre d'avantages qui les rendent très bien adaptés à leur utilisation comme liquides diélectriques: très grande stabilité à la température et à l'hydrolyse, inflammabilité nulle ou très faible, tension de vapeur faible, prix peu élevé. En revanche, leur absence de biodégradabilité conduit à leur accumulation dans l'environnement, ce qui restreint considérablement leur champ d'application et a amené certains pays à les interdire de façon totale ou partielle. De plus, à très basse température, leurs propriétés diélectriques accusent une chute rapide ce qui rend difficile leur utilisation dans des conditions de froid extrême.

D'autres produits ont également été proposés comme liquides diélectriques, par exemple les esters décrits dans le BF n° 2 322 435, mais ces esters sont facilement combustibles. Quant aux polychloropolyphénylalcanes décrits dans le BF n° 2 273 351, ils sont fabriqués à partir de dihalogénures d'alcanes, par exemple le dichloro-1-1 éthane, ce qui sur le plan économique ne présente pas d'intérêt.

Le BF 931 987 propose comme produits diélectriques des diphénylméthanes chlorés dont la composition est mal définie ce qui est dû en particulier à un procédé de fabrication dont les conditions ne peuvent être maîtrisées. Quant au BF 2 312 841, il décrit des diphénylméthanes halogénés ayant obligatoirement un groupe phényle non substitué, condition indispensable d'après ce brevet pour assurer la biodégradabilité des produits. Le brevet belge 402 381 divulgue des produits ayant plusieurs noyaux aromatiques liés chacun à un groupement aliphatique, et plus précisément, de dérivés polyphénylés du méthane, halogénés dans un noyau aromatique au moins. Cependant, il n'est pas question dans cette définition de substituants autres qu'halogénés.

Or, la demanderesse a découvert qu'il était possible d'obtenir, à partir d'une matière première simple et peu coûteuse — les chlorotoluènes ou les chloroxylènes — des diphénylméthanes chlorés et substitués sur un ou deux noyaux, ne possédant pas le noyau biphényle et ayant des propriétés leur permettant de se substituer avantageusement aux polychlorobiphényles dans leurs applications diélectriques sans en présenter les inconvénients. En effet, l'absence de noyaux biphényles et la présence de groupements alkyles sur les noyaux aromatiques a un effet favorable sur la biodégradabilité (voir à ce sujet l'article de G. Sundstrom, K. Olie et O. Hutzinger, Chemosphère n° 213 pages 103—109, 1977).

Il a été découvert, de plus, que ces produits avaient, par comparaison avec les chlorobiphényles, des propriétés électriques très améliorées aux températures très basses, ce qui les rend particulièrement utiles dans les conditions extrêmes d'utilisation.

La demanderesse a également mis en lumière les propriétés fondamentales de ces composés:

— un moment dipolaire élevé,
— une bonne stabilité chimique,

que ces produits soient soumis à des traitements chimiques ou à l'action d'un champ électrique intense.

— une bonne résistance aux inflammations ou aux incendies, ce qui fait d'eux des constituants de choix pour des fluides isolants utilisables en particulier pour les transformateurs.

Ces propriétés se traduisent par une valeur de constante diélectrique (ou permittivité) élevée dans une large zone de température qui couvre l'intervalle normal d'utilisation des liquides diélectriques, et par une valeur très basse du facteur de pertes.

Les produits selon l'invention peuvent être représentés par la formule générale suivante:

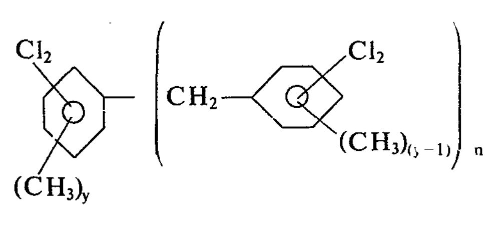

dans laquelle n = 1 ou 2
y = 1 ou 2

Lorsque y = 1, ce sont des chlorotoluènes qui ont été mis en jeu, le cas de y = 2 correspond à l'emploi de chloroxylènes.

Ils sont préparés à partir d'un isomère de chlorotoluène ou de chloroxylène ou d'un mélange d'isomères que l'on soumet à une chloration radicalaire initiée de manière classique, c'est-à-dire, soit photochimiquement, soit en présence d'un initiateur radicalaire. Cette réaction peut être faite à des températures comprises entre 0 et 150° C, et de préférence entre 20 et 100° C. Le mélange ainsi obtenu est alors additionné de catalyseur de Friedel Crafts, par exemple $AlCl_3$, $AlBr_3$, $FeCl_3$. Il se produit une condensation entre les chlorures de chlorobenzyles ou de méthylchlorobenzyles formés et les chlorotoluènes ou chloroxylènes en excès selon la réaction suivante:

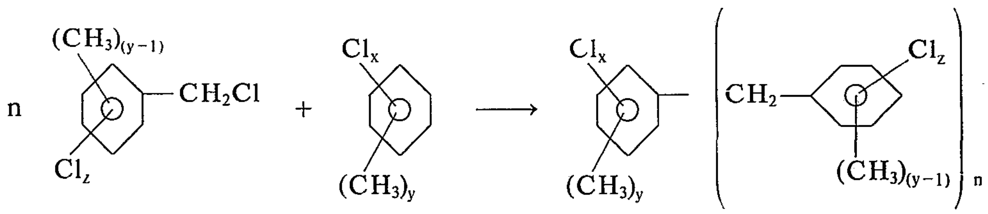

Cette réaction peut se faire entre 20 et 100° C, et suivant l'excès de chlorotoluènes ou chloroxylènes présents, la proportion de produit dans lequel n = 1 est plus ou moins grande, un excès important de chlorotoluènes ou chloroxylènes favorisant l'obtention d'un produit où n = 1. Après destruction du catalyseur et lavage de la phase organique, on soumet le mélange à une distillation et l'on récupère les chlorotoluènes ou chloroxylènes n'ayant pas réagi, qui peuvent être recyclés dans une opération ultérieure. Le mélange de produit où n = 1 ou 2 est alors séparé par distillation.

Bien entendu, les composés bruts de la réaction doivent être soumis à un traitement préliminaire de purification, avec des alcalins (NaOH, $Na_2CO_3$, $NaHCO_3$, ou des composés analogues du calcium ou du potassium), à une température pouvant aller de 20 à 350° C, de préférence entre 200 et 250° C, pendant un temps qui varie selon la température choisie. Parfois, une distillation ultérieure peut être avantageuse.

Après ce traitement préliminaire, la phase de purification suivante consiste à employer une terre de foulon ou d'alumine activée, soit seules, soit en mélange, selon les techniques spécifiques connues dans le secteur des liquides diélectriques.

De même, il peut être avantageux d'ajouter des stabilisants du type époxyde ou d'autre nature comme par exemple le tétraphénylétain ou des composés anthraquinoniques employés en courant continu.

Ces adjuvants sont généralement des accepteurs d'acide chlorhydrique et sont ajoutés en quantités variables entre 0,001 et 10%, de préférence entre 0,01 et 0,3%.

Par ailleurs, si le liquide diélectrique est utilisé comme fluide isolant pour transformateurs, il est possible et parfois intéressant de le mélanger, sans préjudice de ses bonnes qualités avec des produits avant la formule générale suivante:

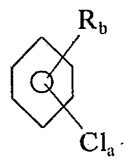

où a = 2 à 4,
b = 0 à 2,

et R est un radical hydrocarboné aliphatique ayant de 1 à 3 atomes de carbone.

Parmi les produits répondant à cette formule générale, on utilise couramment des trichloro ou tétrachloro-benzènes qui présentent une tension de vapeur assez limitée, et qui peuvent être introduits à raison de 30% et jusqu'à 60% en poids du mélange.

Les exemples suivants illustrent l'invention sans toutefois la limiter.

## Exemple 1

Dans un réacteur de 6 litres muni d'une agitation, d'un tube d'alimentation de chlore et d'une lampe actinique Philips TLADK de 30 W, on place un mélange d'isomères de dichlorotoluène (40 moles). On introduit en 4 heures 568 g de chlore gazeux (8 moles) en maintenant la température à 70° C. Le produit de la réaction est alors soutiré du réacteur sauf 100 ml dans lesquels on ajoute 16 g de $FeCl_3$. Le produit soutiré est alors additionné en 2 heures en maintenant la température à 30° C. En fin de réaction, on

chauffe durant 15 minutes à 100°C. Le produit obtenu est lavé sous agitation par 1 litre d'acide chlorhydrique aqueux à 10% puis par deux fois un litre d'eau. La phase organique ainsi obtenue est distillée sous un vide de 13 mm de mercure, la fraction principale distillant entre 230 et 255°C étant constituée par un mélange d'isomères correspondant à la formule générale avec n = 1. Le produit ainsi préparé a une densité à 20°C de 1,372 et une teneur en chlore de 43,9% (théorie = 44,4%).

Exemple 2

6,8 kg de dichlorotoluènes (obtenus par chloration limitée de toluène en présence de $FeCl_3$ à 50°C) sont placés dans un réacteur muni d'un agitateur, d'un dispositif de refroidissement et de chauffage et d'un dispositif d'introduction pour le chlore, d'un dispositif de dégagement d'acide chlorhydrique à travers un réfrigérant et d'une lampe actinique. La température est portée à 80°C et l'on introduit du chlore à raison de 100 g/h. Au bout de cinq heures, la réaction est arrêtée. Le mélange est dégazé par de l'azote et transvasé dans une ampoule de coulée, à part 500 ml environ dans lesquels on ajoute 16 g de $FeCl_3$. Le produit soutiré est alors réintroduit en 2 heures dans le réacteur en maintenant la température à 80–100°C. Il se produit un abondant dégagement d'acide chlorhydrique. On maintient la température durant 2 heures supplémentaires. Le mélange obtenu est lavé à l'eau chaude puis froide et les dichlorotoluènes en excès sont distillés sur une colonne de 30 plateaux sous une pression de 250 mm Hg, que l'on abaisse ensuite à 10 mm de Hg. On isole une fraction de 2240 g de produit de point d'ébullition compris entre 210 et 240°C. Ce produit contient 44,3% de chlore et correspond à la formule:

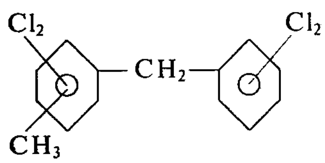

On ajoute à la fraction ainsi recueillie, 1833 g (soit 45% du mélange obtenu) de trichlorobenzène commercial constitué par un mélange des isomères suivants:

| | |
|---|---|
| 1,2,3-trichlorobenzène | 36% |
| 1,2,4-trichlorobenzène | 63% |
| tétrachlorobenzène | 1% |

on ajoute 4 g de tétraphénylétain. Après purification diélectrique classique par traitements successifs sur alumine activée, on mesure les propriétés physiques et diélectriques du mélange, que l'on compare avec celles d'un mélange de 70% d'Askarel (mélange classique de polychlorobiphényle) et 30% de trichlorobenzène.

| | Mélange suivant exemple | Askarel type D |
|---|---|---|
| Densité à 20°C | 1,409 | |
| Point de ramollissement | <–55°C | <–35°C |
| Viscosité à 20°C | 8,1 cst | 18–20 cst |
| Point éclair | 130–145°C | 130–145°C |
| Point de feu | néant | néant |
| Rigidité diélectrique | 280 KV/cm | >200 KV/cm |
| Résistivité (500 V) (90°C) | $10^{12}\Omega$ x cm | $>10^{12}\Omega$ x cm |
| Tg $\delta$ x 100 (90° – 50 Hz) | <5 | <2 |
| Constante diélectrique (90° – 50 Hz) | 4,4 | 4,0 à 4,1 |

Les figures 1 et 2 montrent les différences entre le mélange obtenu dans cet exemple et l'Askarel type D en ce qui concerne le facteur de pertes en fonction de la température et de la fréquence et les figures 3 et 4 les différences entre les constantes diélectriques en ce qui concerne les mêmes produits.

## Revendications

1. Nouveaux diélectriques liquides de formule:

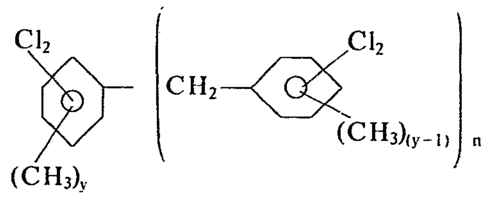

(1)

où n et y ont la valeur 1 ou 2.

2. Procédé de préparation des produits de la revendication 1 par chloration radicalaire partielle d'un isomère ou d'un mélange d'isomères de chlorotoluène ou de chloroxylène ou d'un mélange des deux, suivie d'une condensation en présence d'un catalyseur de Friedel-Crafts entre les chlorures de chlorobenzyle ou de méthylchlorobenzyle formés et les chlorotoluènes ou chloroxylènes en excès.

3. Utilisation des produits diélectriques liquides de la revendication 1 comme fluides isolants pour transformateurs, ou comme matériau isolant utilisé tel quel par imprégnation entre les lames d'un condensateur.

4. Utilisation comme fluides isolants pour transformateurs des produits de la revendication 1 en mélange avec un ou plusieurs produits de formule générale:

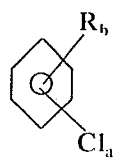

(2)

où a varie de 2 à 4, b varie de 0 à 2, et R est un radical hydrocarboné aliphatique comportant de 1 à 3 atomes de carbone, ce mélange étant additionné d'un accepteur d'acide du type huile époxydée ou tétraphénylétain en quantité comprise entre 0,001 et 10% et de préférence de 0,01 à 0,3%.

5. Utilisation comme fluides isolants pour transformateurs de mélanges selon la revendication 4, où le produit de la revendication 1 est le produit suivant:

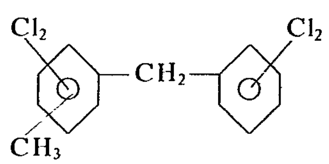

où $n = y = 1$

introduit à raison de 40 à 70% dans du trichlorobenzène commercial de composition suivante:

isomère 1-2-4 = 50 à 85%
isomère 1-2-3 = 50 à 15%

## Patentansprüche

1. Neue flüssige Dielektrika der Formel:

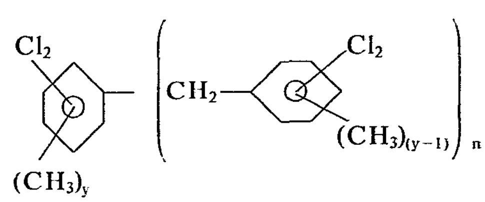

in der n und y den Wert 1 oder 2 haben.

2. Verfahren zur Herstellung der Mittel nach Anspruch 1 durch partielle radikalische Chlorierung eines Isomeren oder eines Isomerengemisches von Chlortoluol, Chlorxylol oder einem Gemisch von beiden, gefolgt von einer Kondensation der gebildeten Chloride des Benzylchlorids oder des Methylbenzylchlorids mit den überschüssigen Chlortoluolen oder Chlorxylolen in Gegenwart eines Friedel-Crafts-Katalysators.

3. Verwendung der flüssigen Dielektrika nach Anspruch 1 als Isolierflüssigkeit für Transformatoren oder als Isoliermaterial, das als solches zum Imprägnieren zwischen den Lamellen eines Kondensators verwendet wird.

4. Verwendung der Mittel nach Anspruch 1 als Isolierflüssigkeit für Transformatoren im Gemisch mit einem oder mehreren Stoffen der allgemeinen Formel:

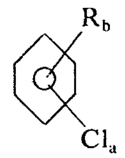

in der a von 2 bis 4 und b von 0 bis 2 reicht und R ein aliphatischer Kohlenwasserstoffrest mit 1 bis 3 Kohlenstoffatomen ist, wobei dem Gemisch ein Säureakzeptor vom Typ epoxydierten Öls oder Tetraphenylzinn in einer Menge zwischen 0,01 und 10%, vorzugsweise von 0,01 bis 0,3% zugegeben ist.

5. Verwendung der Gemische nach Anspruch 4 als Isolierflüssigkeit für Transformatoren, wobei das Mittel des Anspruchs 1 die folgende Verbindung mit n = y = 1 ist:

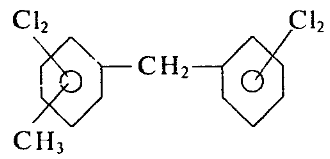

die im Verhältnis von 40 bis 70% in handelsüblichem Trichlorbenzol der Zusammensetzung

1-2-4-Isomeres = 50 bis 85%
1-2-3-Isomeres = 50 bis 15%

eingesetzt wird.

**Claims**

1. New liquid dielectrics of the formula:

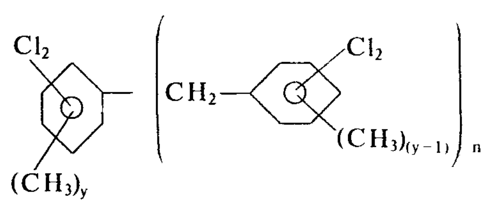

(1)

in which n and y have the value 1 or 2.

2. Process for the preparation of the products of Claim 1, by the partial free-radical chlorination of an isomer or a mixture of isomers of chlorotoluene or chloroxylene or a mixture of the two, followed by condensation, in the presence of a Friedel-Crafts catalyst, of the chlorobenzyl or methylchlorobenzyl chlorides formed and the excess chlorotoluenes or chloroxylenes.

3. Use of the liquid dielectric products of Claim 1 as insulating fluids for transformers, or as an insulating material used as such by impregnation between the plates of a capacitor.

4. Use, as insulating fluids for transformers, of the products of Claim 1 mixed with one or more products of the general formula:

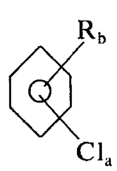

(2)

in which a varies form 2 to 4, b varies from 0 to 2 and R is an aliphatic hydrocarbon radical containing from 1 to 3 carbon atoms, an acid acceptor of the epoxidised oil type or tetraphenyltin type being added to this mixture in an amount of between 0.001 and 10% and preferably of 0.01 to 0.3%.

5. Use, as insulating fluids for transformers, of mixtures according to Claim 4 in which the product of Claim 1 is the following product:

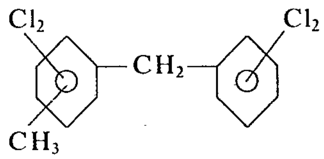

in which n = y = 1, introduced in an amount of 40 to 70% into commercial trichlorobenzene of the following composition:

1,2,4-isomer = 50 to 85%
1,2,3-isomer = 50 to 15%.

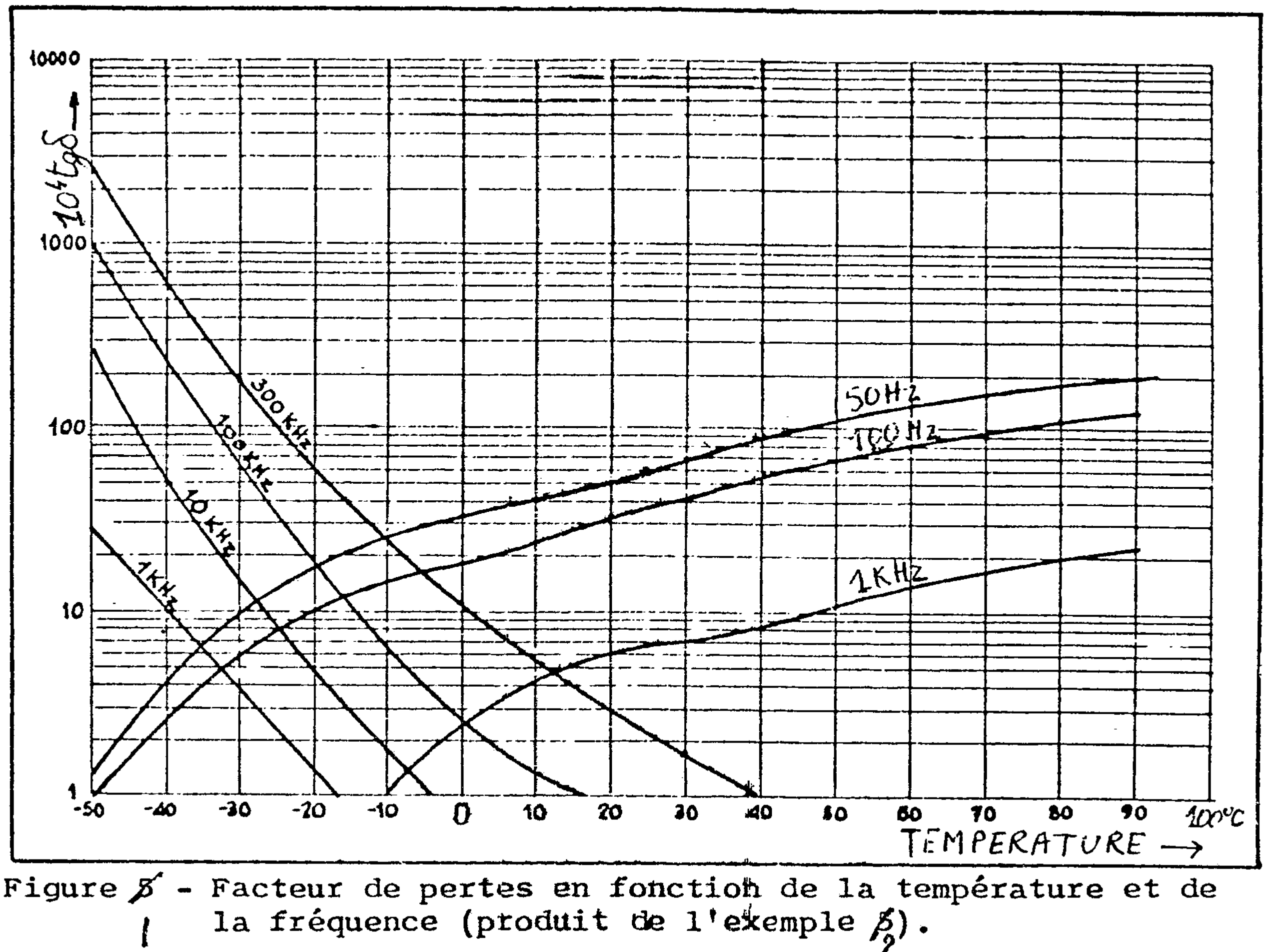


Figure 1 - Facteur de pertes en fonction de la température et de la fréquence (produit de l'exemple 2).

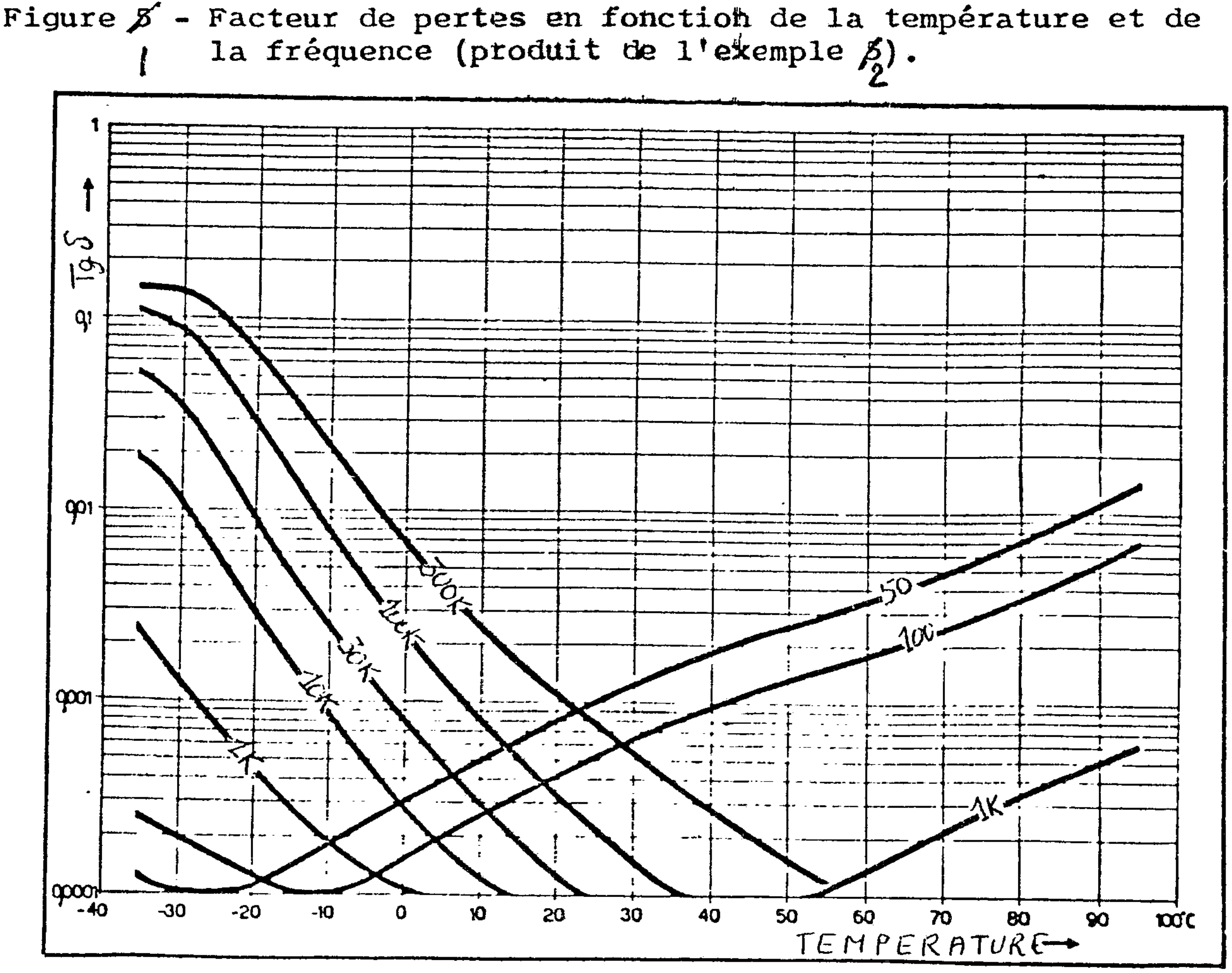


Figure 2 - Facteur de pertes en fonction de la température et de la fréquence (Askarel type D).

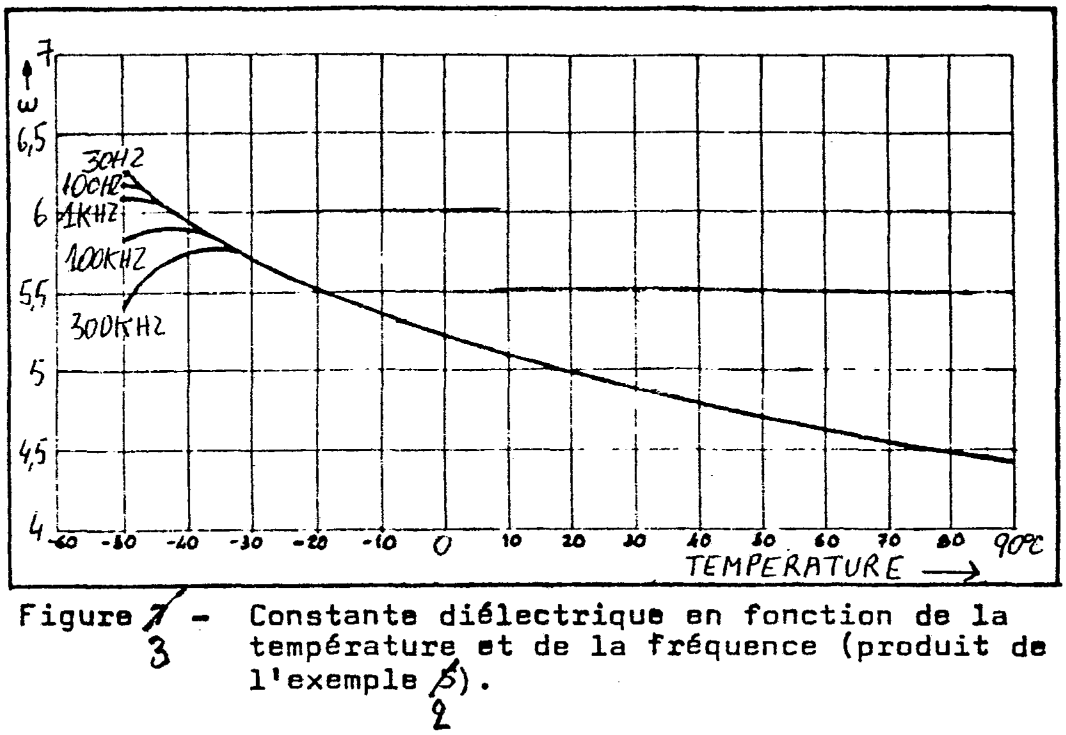


Figure ~~7~~ 3 – Constante diélectrique en fonction de la température et de la fréquence (produit de l'exemple ~~5~~ 2).

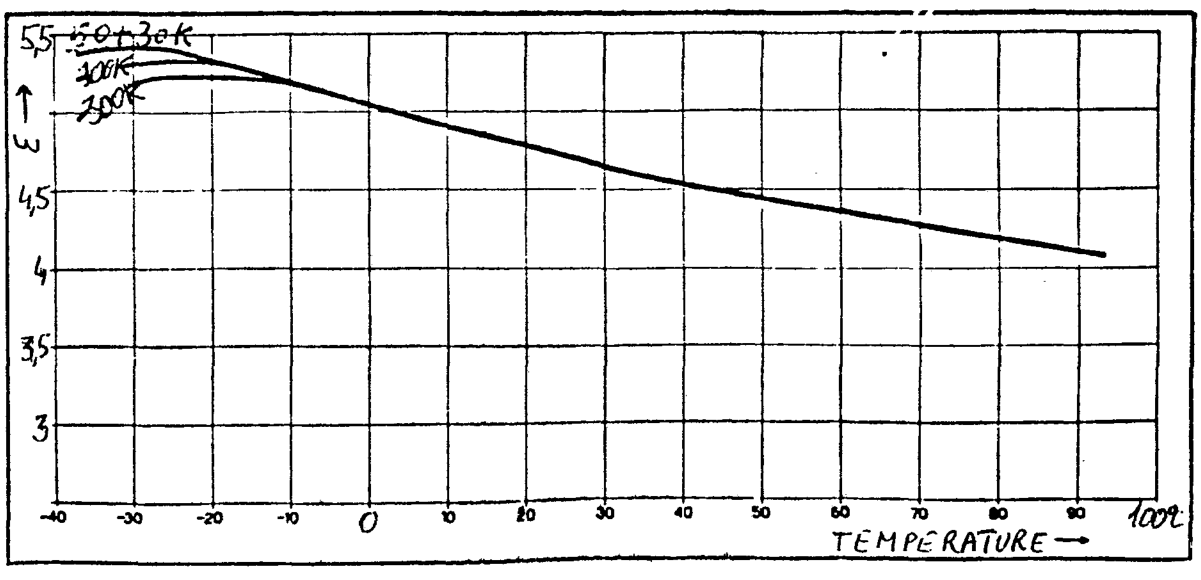


Figure ~~8~~ 4 – Constante diélectrique en fonction de la température et de la fréquence (Askarel type D).